# EUROPEAN PATENT APPLICATION

(11) **EP 1 586 587 A1**
(43) Date of publication of application: **19.10.2005**
(21) Application number: 04291020.8
(22) Date of filing: 16.04.2004
(51) Int. Cl.: C07K 14/81, C07K 16/38, C12Q 1/68, G01N 33/574, G01N 33/68

(54) **Compositions and methods for detecting angiogenesis**

(71) Applicant: Exonhit Therapeutics SA, 75017 Paris (FR)
(72) Inventor: Kearsey, Jonathan, 91210 Draveil (FR)
(74) Representative: Becker, Philippe

(57) **Abstract**

The present application pertains to the field of biology and health. It is more particularly related to the identification and functional characterisation of a new protein involved in angiogenic mechanisms, as well as the use of this protein as a diagnostic, therapeutic or screening target. It also relates to the tools that can be used for carrying out diagnostic, therapeutic or screening methods, such as, particularly probes, antibodies, vectors, recombinant cells, etc. The invention is particularly useful for preparing pharmaceutical compounds for the fields of cancer, angiogenesis, inflammatory diseases or tissue changes.

SEQUENCE LISTING

<110>  Exonhit Therapeutics SA

<120>  COMPOSITIONS AND METHODS FOR DETECTING ANGIOGENESIS

<130>  B0277EP

<160>  17

<170>  PatentIn version 3.1

<210>  1
<211>  1544
<212>  DNA
<213>  Homo sapiens

<220>
<221>  misc_feature
<222>  (1538)..(1538)
<223>  n means any base

<220>
<221>  misc_feature
<222>  (1358)..(1358)
<223>  n means any base

<400>  1
gcacgagttg ggaggtgtag cgcggctctg aacgcgctga gggccgttga gtgtcgcagg      60
cggcgagggc gcgagtgagg agcagaccca ggcatcgcgc gccgagaagg ccgggcgtcc     120
ccacactgaa ggtccggaaa ggcgacttcc gggggctttg gcacctggcg gaccctcccg     180
gagcgtcggc acctgaacgc gaggcgctcc attgcgcgtg cgcgttgagg ggcttcccgc     240
acctgatcgc gagaccccaa cggctggtgg cgtcgcctgc gcgtctcggc tgagctggcc     300
atggcgcagc tgtgcgggct gaggcggagc cgggcgtttc tcgccctgct gggatcgctg     360
ctcctctctg gggtcctggc ggccgaccga gaacgcagca tccacgactt ctgcctggtg     420

B0277-HAI-2.ST25.txt
tcgaaggtgg tgggcagatg ccgggcctcc atgcctaggt ggtggtacaa tgtcactgac 480
ggatcctgcc agctgtttgt gtatgggggc tgtgacggaa acagcaataa ttacctgacc 540
aaggaggagt gcctcaagaa atgtgccact gtcacagaga atgccacggg tgacctggcc 600
accagcagga atgcagcgga ttcctctgtc ccaagtgctc ccagaaggca ggattctgaa 660
gaccactcca gcgatatgtt caactatgaa gaatactgca ccgccaacgc agtcactggg 720
ccttgccgtg catccttccc acgctggtac tttgacgtgg agaggaactc ctgcaataac 780
ttcatctatg gaggctgccg gggcaataag aacagctacc gctctgagga ggcctgcatg 840
ctccgctgct tccgccagca ggagaatcct cccctgcccc ttggctcaaa ggtggtggtt 900
ctggcggggc tgttcgtgat ggtgttgatc ctcttcctgg gagcctccat ggtctacctg 960
atccgggtgg cacggaggaa ccaggagcgt gccctgcgca ccgtctggag ctccggagat 1020
gacaaggagc agctggtgaa gaacacatat gtcctgtgac cgccctgtcg ccaagaggac 1080
tggggaaggg aggggagact atgtgtgagc tttttttaaa tagagggatt gactcggatt 1140
tgagtgatca ttagggctga ggtctgtttc tctgggaggt aggacggctg cttcctggtc 1200
tggcagggat gggtttgctt tggaaatcct ctaggaggct cctcctcgca tggcctgcag 1260
tctggcagca gccccgagtt gtttcctcgc tgatcgattt ctttcctcca ggtagagttt 1320
tctttgctta tgttgaattc cattgcctcc ttttctcnat cacagaagtg atgttggaat 1380
cgtttctttt gtttgtctga tttatggttt ttttaagtat aaacaaaagt tttttattag 1440
cattctgaaa gaaggaaagt aaaatgtaca agtttaataa aaaggggcct tcccctttag 1500
aataaatttc cagcatgttg ctttcaaaaa aaaaaaaaaa aaaa 1544

<210> 2
<211> 1373
<212> DNA
<213> Homo sapiens

<220>
<221> misc_feature
<222> (1187)..(1187)
<223> n means any base

<400> 2
gcacgagttg ggaggtgtag cgcggctctg aacgcgctga gggccgttga gtgtcgcagg 60
cggcgagggc gcgagtgagg agcagaccca ggcatcgcgc gccgagaagg ccgggcgtcc 120
ccacactgaa ggtccggaaa ggcgacttcc gggggctttg gcacctggcg gaccctcccg 180
gagcgtcggc acctgaacgc gaggcgctcc attgcgcgtg cgcgttgagg ggcttcccgc 240

B0277-HAI-2.ST25.txt
acctgatcgc gagaccccaa cggctggtgg cgtcgcctgc gcgtctcggc tgagctggcc 300
atggcgcagc tgtgcgggct gaggcggagc cgggcgtttc tcgccctgct gggatcgctg 360
ctcctctctg gggtcctggc ggccgaccga gaacgcagca tccacgagaa tgccacgggt 420
gacctggcca ccagcaggaa tgcagcggat tcctctgtcc caagtgctcc cagaaggcag 480
gattctgaag accactccag cgatatgttc aactatgaag aatactgcac cgccaacgca 540
gtcactgggc cttgccgtgc atccttccca cgctggtact ttgacgtgga gaggaactcc 600
tgcaataact tcatctatgg aggctgccgg ggcaataaga acagctaccg ctctgaggag 660
gcctgcatgc tccgctgctt ccgccagcag gagaatcctc ccctgcccct tggctcaaag 720
gtggtggttc tggcggggct gttcgtgatg gtgttgatcc tcttcctggg agcctccatg 780
gtctacctga tccgggtggc acggaggaac caggagcgtg ccctgcgcac cgtctggagc 840
tccggagatg acaaggagca gctggtgaag aacacatatg tcctgtgacc gccctgtcgc 900
caagaggact ggggaaggga ggggagacta tgtgtgagct ttttttaaat agagggattg 960
actcggattt gagtgatcat tagggctgag gtctgtttct ctgggaggta ggacggctgc 1020
ttcctggtct ggcagggatg ggtttgcttt ggaaatcctc taggaggctc ctcctcgcat 1080
ggcctgcagt ctggcagcag ccccgagttg tttcctcgct gatcgatttc tttcctccag 1140
gtagagtttt ctttgcttat gttgaattcc attgcctcct tttctcnatc acagaagtga 1200
tgttggaatc gtttcttttg tttgtctgat ttatggtttt tttaagtata aacaaaagtt 1260
ttttattagc attctgaaag aaggaaagta aaatgtacaa gtttaataaa aaggggcctt 1320
cccctttaga ataaatttcc agcatgttgc tttcaaaaaa aaaaaaaaaa aaa 1373

<210> 3
<211> 1414
<212> DNA
<213> Homo sapiens

<220>
<221> misc_feature
<222> (1228)..(1228)
<223> n means any base

<400> 3
gcgagtgagg agcagaccca ggcatcgcgc gccgagaagg ccgggcgtcc ccacactgaa 60
ggtccggaaa ggcgacttcc gggggctttg gcacctggcg gaccctcccg gagcgtcggc 120
acctgaacgc gaggcgctcc attgcgcgtg cgcgttgagg ggcttcccgc acctgatcgc 180
gagaccccaa cggctggtgg cgtcgcctgc gcgtctcggc tgagctggcc atggcgcagc 240

B0277-HAI-2.ST25.txt

tgtgcgggct gaggcggagc cgggcgtttc tcgccctgct gggatcgctg ctcctctctg 300

gggtcctggc ggccgaccga gaacgcagca tccacgactt ctgcctggtg tcgaaggtgg 360

tgggcagatg ccgggcctcc atgcctaggt ggtggtacaa tgtcactgac ggatcctgcc 420

agctgtttgt gtatgggggc tgtgacggaa acagcaataa ttacctgacc aaggaggagt 480

gcctcaagaa atgtgccact gtcacagctc ccagaaggca ggattctgaa gaccactcca 540

gcgatatgtt caactatgaa gaatactgca ccgccaacgc agtcactggg ccttgccgtg 600

catccttccc acgctggtac tttgacgtgg agaggaactc ctgcaataac ttcatctatg 660

gaggctgccg gggcaataag aacagctacc gctctgagga ggcctgcatg ctccgctgct 720

tccgccagca ggagaatcct cccctgcccc ttggctcaaa ggtggtggtt ctggcggggc 780

tgttcgtgat ggtgttgatc ctcttcctgg gagcctccat ggtctacctg atccgggtgg 840

cacggaggaa ccaggagcgt gccctgcgca ccgtctggag ctccggagat gacaaggagc 900

agctggtgaa gaacacatat gtcctgtgac cgccctgtcg ccaagaggac tggggaaggg 960

aggggagact atgtgtgagc tttttttaaa tagagggatt gactcggatt tgagtgatca 1020

ttagggctga ggtctgtttc tctgggaggt aggacggctg cttcctggtc tggcagggat 1080

gggtttgctt tggaaatcct ctaggaggct cctcctcgca tggcctgcag tctggcagca 1140

gccccgagtt gtttcctcgc tgatcgattt ctttcctcca ggtagagttt tctttgctta 1200

tgttgaattc cattgcctcc ttttctcnat cacagaagtg atgttggaat cgtttctttt 1260

gtttgtctga tttatggttt ttttaagtat aaacaaaagt tttttattag cattctgaaa 1320

gaaggaaagt aaaatgtaca agtttaataa aaaggggcct tcccctttag aataaatttc 1380

cagcatgttg ctttcaaaaa aaaaaaaaaa aaaa 1414

<210> 4

<211> 252

<212> PRT

<213> Homo sapiens

<400> 4

<210>  5

<211>  195

<212>  PRT

<213>  Homo sapiens


<400>  5

Met Ala Gln Leu Cys Gly Leu Arg Arg Ser Arg Ala Phe Leu Ala Leu
1               5                   10                  15

Leu Gly Ser Leu Leu Leu Ser Gly Val Leu Ala Ala Asp Arg Glu Arg
            20                  25                  30

B0277-HAI-2.ST25.txt

Ser Ile His Asp Asn Ala Thr Gly Asp Leu Ala Thr Ser Arg Asn Ala
        35                  40                  45

Ala Asp Ser Ser Val Pro Ser Ala Pro Arg Arg Gln Asp Ser Glu Asp
    50                  55                  60

His Ser Ser Asp Met Phe Asn Tyr Glu Glu Tyr Cys Thr Ala Asn Ala
65                  70                  75                  80

Val Thr Gly Pro Cys Arg Ala Ser Phe Pro Arg Trp Tyr Phe Asp Val
                85                  90                  95

Glu Arg Asn Ser Cys Asn Asn Phe Ile Tyr Gly Gly Cys Arg Gly Asn
            100                 105                 110

Lys Asn Ser Tyr Arg Ser Glu Glu Ala Cys Met Leu Arg Cys Phe Arg
        115                 120                 125

Gln Gln Glu Asn Pro Pro Leu Pro Leu Gly Ser Lys Val Val Val Leu
    130                 135                 140

Ala Gly Leu Phe Val Met Val Leu Ile Leu Phe Leu Gly Ala Ser Met
145                 150                 155                 160

Val Tyr Leu Ile Arg Val Ala Arg Arg Asn Gln Glu Arg Ala Leu Arg
                165                 170                 175

Thr Val Trp Ser Ser Gly Asp Asp Lys Glu Gln Leu Val Lys Asn Thr
            180                 185                 190

Tyr Val Leu
        195

<210>  6

<211>  232

<212>  PRT

<213>  Homo sapiens


<400>  6

Met Ala Gln Leu Cys Gly Leu Arg Arg Ser Arg Ala Phe Leu Ala Leu
1               5                   10                  15

Leu Gly Ser Leu Leu Leu Ser Gly Val Leu Ala Ala Asp Arg Glu Arg
            20                  25                  30

Ser Ile His Asp Phe Cys Leu Val Ser Lys Val Val Gly Arg Cys Arg
        35                  40                  45

B0277-HAI-2.ST25.txt

Ala Ser Met Pro Arg Trp Trp Tyr Asn Val Thr Asp Gly Ser Cys Gln
    50                  55                  60

Leu Phe Val Tyr Gly Gly Cys Asp Gly Asn Ser Asn Asn Tyr Leu Thr
65                  70                  75                  80

Lys Glu Glu Cys Leu Lys Lys Cys Ala Thr Val Thr Glu Pro Arg Arg
                85                  90                  95

Gln Asp Ser Glu Asp His Ser Ser Asp Met Phe Asn Tyr Glu Glu Tyr
            100                 105                 110

Cys Thr Ala Asn Ala Val Thr Gly Pro Cys Arg Ala Ser Phe Pro Arg
        115                 120                 125

Trp Tyr Phe Asp Val Glu Arg Asn Ser Cys Asn Asn Phe Ile Tyr Gly
    130                 135                 140

Gly Cys Arg Gly Asn Lys Asn Ser Tyr Arg Ser Glu Glu Ala Cys Met
145                 150                 155                 160

Leu Arg Cys Phe Arg Gln Gln Glu Asn Pro Pro Leu Pro Leu Gly Ser
                165                 170                 175

Lys Val Val Val Leu Ala Gly Leu Phe Val Met Val Leu Ile Leu Phe
            180                 185                 190

Leu Gly Ala Ser Met Val Tyr Leu Ile Arg Val Ala Arg Arg Asn Gln
        195                 200                 205

Glu Arg Ala Leu Arg Thr Val Trp Ser Ser Gly Asp Asp Lys Glu Gln
    210                 215                 220

Leu Val Lys Asn Thr Tyr Val Leu
225                 230

<210>  7

<211>  294

<212>  DNA

<213>  Homo sapiens

<400>  7
gttaccaagg caaagatgga tgagggcatc tcaggagaga ctacttgtta tgattcttga      60

gttttgacca acagaaatga acttggacca acttaagcaa ggaaaaagcg ttcatgggaa     120

ggatggtggg atagctcaca gagaatgcca cgggtgacct ggccaccaac ataaatgcag     180

aggattcctc tgtcccaagt gctcccagaa ggcaggattc tgaagaccac tccagcgata     240

B0277-HAI-2.ST25.txt
tgttcaacta tgaagaatac tgcaccgcca acgcagtcac tgggactgga cccg          294

<210>  8

<211>  30

<212>  DNA

<213>  Homo sapiens


<400>  8
aacgcagcat ccacgacttc tgcctggtgt                                      30

<210>  9

<211>  30

<212>  DNA

<213>  Homo sapiens


<400>  9
gtgccactgt cacagagaat gccacgggtg                                      30

<210>  10

<211>  30

<212>  DNA

<213>  Homo sapiens


<400>  10
aacgcagcat ccacgagaat gccacgggtg                                      30

<210>  11

<211>  30

<212>  DNA

<213>  Homo sapiens


<400>  11
cctctgtccc aagtgctccc agaaggcagg                                      30

<210>  12

<211>  30

<212>  DNA

<213>  Homo sapiens

B0277-HAI-2.ST25.txt

<400>  12
gtgccactgt cacagctccc agaaggcagg                                        30

<210>  13

<211>  30

<212>  PRT

<213>  Homo sapiens

<400>  13

Leu Ser Gly Val Leu Ala Ala Asp Arg Glu Arg Ser Ile His Asp Phe
1               5                   10                  15

Cys Leu Val Ser Lys Val Val Gly Arg Cys Arg Ala Ser Met
            20                  25                  30

<210>  14

<211>  30

<212>  PRT

<213>  Homo sapiens

<400>  14

Leu Thr Lys Glu Glu Cys Leu Lys Lys Cys Ala Thr Val Thr Glu Asn
1               5                   10                  15

Ala Thr Gly Asp Leu Ala Thr Ser Arg Asn Ala Ala Asp Ser
            20                  25                  30

<210>  15

<211>  30

<212>  PRT

<213>  Homo sapiens

<400>  15

Leu Ser Gly Val Leu Ala Ala Asp Arg Glu Arg Ser Ile His Asp Asn
1               5                   10                  15

Ala Thr Gly Asp Leu Ala Thr Ser Arg Asn Ala Ala Asp Ser
            20                  25                  30

<210>  16

B0277-HAI-2.ST25.txt
<211>  30

<212>  PRT

<213>  Homo sapiens


<400>  16

Leu Ala Thr Ser Arg Asn Ala Ala Asp Ser Ser Val Pro Ser Ala Pro
1               5                   10                  15

Arg Arg Gln Asp Ser Glu Asp His Ser Ser Asp Met Phe Asn
            20                  25                  30

<210>  17

<211>  30

<212>  PRT

<213>  Homo sapiens


<400>  17

Leu Thr Lys Glu Glu Cys Leu Lys Lys Cys Ala Thr Val Thr Glu Pro
1               5                   10                  15

Arg Arg Gln Asp Ser Glu Asp His Ser Ser Asp Met Phe Asn
            20                  25                  30

## Description

The present application pertains to the field of biology and health. It is more particularly related to the identification and characterization of a novel protein involved in hypoxic and angiogenic mechanisms, as well as the use of this protein as a diagnostic, therapeutic or screening target. It also relates to tools that can be used for carrying out diagnostic, therapeutic or screening methods, such as, particularly, probes, antibodies, vectors, recombinant cells, and the like. The invention may be used to treat or diagnose various diseases in human subjects, including cancers, as well as to develop therapeutic compounds suitable to treat or diagnose such diseases.

Cellular responses to hypoxia have important effects on the development and metastasis of tumors, angiogenesis, wound healing, recovery from ischemia, and other physiological and pathological processes. Reduced oxygen availability can trigger a variety of cellular mechanisms including angiogenesis, cell-cycle arrest, apoptosis, and glycolysis.

Angiogenesis is a complex process by which new blood vessels are formed, typically from pre-existing vasculature. Angiogenesis mainly takes place during the course of embryonic development, but also has an essential role in the adult stage in certain highly regulated physiological processes, such as, notably during the processes of wound healing, ovulation and menstruation.

Normal tissues maintain a balance between cellular proliferation and oxygen supply. This balance is altered in solid tumors, resulting in focal regions with reduced oxygen levels compared to surrounding normal tissue. The cells in hypoxic regions either adapt to the hypoxic stress or die. Adaptation to a low oxygen environment can have serious consequences. For example, hypoxic tumor cells have a higher resistance to radiotherapy and certain chemotherapies. Hypoxia can promote a higher mutation rate and select for a more metastatic and malignant phenotype.

Many diseases have been described as having a component or a stage connected with the phenomenon of angiogenesis. These include the following diseases: many types of cancers, ophthalmological problems such as retinopathies (especially diabetic retinopathy and macular degeneration), atherosclerosis, arthritis, rheumatoid arthritis, psoriasis, or diseases associated with delayed healing. In the case of cancers, it has been established that angiogenesis is not only essential for tumour growth, but that it is also involved in the progression from benign to invasive stage of cancers, and in the progression from dormant metastases to metastatic lesions.

Therefore, inhibition of angiogenesis is thought to provide an opportunity for therapy of cancer and other conditions involving responses to hypoxia. For example, tumor angiogenesis may be blocked by disrupting the expression of VEGF or its receptor.

The present invention now describes the identification of a protein involved in the response to hypoxia. An original approach has enabled the identification of a cDNA corresponding to a portion of the HAI-2 (SPINT2, Bikunin placental) protein, as well as modified forms of this protein, under hypoxic conditions.

The present application for the first time demonstrates that the expression of the HAI-2 protein has biological properties that are important in the regulation of hypoxia and angiogenesis. It particularly shows deregulation of the expression of this protein in response to hypoxic stress, in which the regulation of angiogenesis is important.

This protein thus provides a new basis for diagnostic and therapeutic approaches for diseases involving angiogenic phenomena and, more generally, proliferative processes.

More particularly, a qualitative differential analysis was performed using RNA extracts from a renal cell carcinoma cell line (RCC4). This cell line has an inactive Von Hippel Lindau tumour suppressor gene and will be referred herein as "RCC4-VHL". A second cell line has been derived from RCC4 by transfection with the wild type VHL gene and will be referred herein as "RCC4+VHL". The Von Hippel Lindau (VHL) tumor suppressor gene was isolated in 1993 as the gene that causes the inherited VHL cancer syndrome. Individuals afflicted with VHL disease are at high risk to develop multiple, highly vascularized, tumors of the kidney, brain and eye. VHL patients inherit at conception a mutated, inactivated allele of the VHL tumor suppressor gene. Tumors arise in susceptible cells when the remaining wild-type copy of VHL acquires a somatic mutation. Biallelic inactivating mutations of the VHL gene are also found in sporadic clear cell renal cell carcinoma (RCC), the most common and deadliest form of kidney cancer in human. VHL is a small protein of 213 amino acids that forms a complex with four other proteins referred to as elongin B, elongin C, cullin-2 and rbx-1 (VBC/Cul-2). This complex displays E3-ubiquitin ligase activity, targeting specific proteins for poly-ubiquitination and subsequent degradation by the 26S proteosome. The alpha subunit of hypoxia-inducible factor-1 (HIFα) is a known substrate of VHL-directed ubiquitination and degradation. HIFα are constitutively degraded by the VHL complex in normoxia but stabilized by hypoxia. Under hypoxic conditions, HIF1α is therefore stabilised and induce downstream hypoxic responses including the activation of VEGF.

The pair of cell lines [RCC4+VHL and RCC4-VHL] therefore mimics the role of HIF1α regulation under normoxic and hypoxic conditions, respectively. This allows a comparison of genes induced by HIF1α (RCC4-VHL) as compared to the other cell line where HIF1α protein levels are low (RCC4+VHL). Theses two cell lines were exposed or not exposed to hypoxia (0.1 % O₂, 16 hours). Comparison of RNA from RCC4+VHL grown in normoxic conditions (RCC4+VHL/N) with RNA from RCC4-VHL grown in hypoxic conditions (0.1 % O₂, 16 hours, RCC4-VHL/H) was performed by qualitative differential screening according to the DATAS technique described in patent n° WO99/46403. Using this technique, applicant generated a repertoire of splicing alterations from RCC4 cells induced by hypoxia and focused specifically on the HIF1α and VHL pathways. One of the cDNAs isolated from this DATAS library was derived from the mRNA of the gene encoding the hepatocyte growth factor activator inhibitor 2 protein (HAI-2, also known as serine protease inhibitor, kunitz-type, 2 (SPINT2) or Bikunin, placental. GenBank, under the reference NM_0221102).

The HAI-2/SPINT2 protein is a serine protease inhibitor that inhibits hepatocyte growth factor activator (HGFA). In turn, HGFA controls the activity of hepatocyte growth factor (HGF), through activation of the inactive secreted form of HGF. Over expression of HAI-2 therefore results in inhibition of HFGA and a decrease in the level of circulating active HGF. HGF has been shown to have potent angiogenic activity in a number of tumour, ischemic and neurotrophic models (Bussolino, Di Renzo et al. 1992; Grant, Kleinman et al. 1993). HGF also plays an important role in the regulation of mitogenesis, motogenesis migration and invasion.

The HAI-2 DATAS fragment (ETH-ARCB0803-01) is identical to the Placental Bikunin/Hepatocyte Growth Factor Activator inhibitor Type 2 mRNA (Kawaguchi, Qin et al. 1997), (Delaria, Muller et al. 1997). The HAI-2 cDNA encodes a deduced 252-amino acid protein with a calculated molecular mass of 28.2 kD. HAI-2 contains an N-terminal putative signal peptide sequence, a hydrophobic, membrane associated C-terminal region, and a central region that encodes the processed, cleaved protein. HAI-2 has 2 potential N-glycosylation sites, but a different N-linked sugar chain from that of SPINT1. Two regions show extensive similarity to the Kunitz-type sequence of serine protease inhibitors. Unlike SPINT1, HAI-2 lacks a long N-terminal region preceding the first Kunitz domain and a region that is similar to the ligand-binding domain of the low density lipoprotein receptor between the 2 Kunitz domains. HAI-2 is produced as an active membrane-associated protein and is proteolytically cleaved and secreted. By Northern blot analysis (Kawaguchi, Qin et al. 1997), HAI-2 has been demonstrated to be expressed as a 1.6-kb transcript in adult placenta, kidney, pancreas, prostate, testis, thymus, and trachea. They also showed that HAI-2 is a specific inhibitor of HGF activators. Alternative splicing of exon 2 has been documented for the mouse homologue of HAI-2 (Kataoka et al., 2002 Biochem biophys Res comm.).

The present invention now demonstrates, for the first time, that HAI-2 is deregulated in hypoxic conditions, where angiogenesis is an important process.

Furthermore, using microarray differential expression profiling, a significant differential expression of the mRNA encoding the HAI-2 protein was demonstrated between RNA from the RCC4-VHL and that of the RCC4+VHL cell lines. Differential expression was also observed between RCC4-VHL/H (0.1% O₂, 16 hours) and RCC4+VHL/N (20% O₂, 16 hours). These differential expression data demonstrate, for the first time, that HAI-2 is a hypoxically regulated and under the control of both HIF1α and VHL gene products. Hypoxic regulation of HAI-2 expression can therefore influence the level of HGF activator (HGFa) and, as a consequence, the level of active hepatocyte growth factor (HGF). Hypoxic regulation of HAI-2 provides a mechanism through which HGF-specific mitogenesis, migration and invasion can be controlled.

In addition, bioinformatic analyses and experimental characterisation of HAI-2 species showed the presence of three alternatively spliced mRNA isoforms (SEQ ID NO: 1, 3 and 5). The first one is the wild-type isoform. The second one results in skipping of exon 2 and the third one in skipping of exon 3. The skipping of exon 2 gives rise to an in frame deletion of 57 amino acids, and thereby to the complete deletion of the first Kunitz-type domain. This first Kunitz-type domain of HAI-2 is responsible for the majority of the inhibitory activity of HAI-2 towards HGF/SF activator, at least in the human species (Qin, Denda et al. 1998; Kataoka, Itoh et al. 2002). Thus, this alternative splice event may produce a protein which has a dominant negative effect. The exon 2-skipped mRNA species was never isolated or identified in human subject or samples until the present work was made.

The present invention also experimentally discloses alternative splicing events associated with HAI-2, which result in altered amino acid sequence at the protein level and altered serine protease function related to HAI-2 hypoxic regulation of HGFA and as a consequence HGF.

The present invention also experimentally discloses the increased expression of HAI-2 proteins in colon cancer tissues as compared to matched normal colon tissues, indicating that the HAI-2 proteins could form the basis of a diagnostic assay for the detection of colon cancer.

As the HAI-2 protein and all of its described isoforms retain a signal peptide sequence, the hypoxic regulation of this gene can be monitored by ELISA using whole blood. The present application thus provides a new hypoxically regulated diagnostic target that can be monitored in blood or other biological fluids.

The present application thus provides a new molecular target for angiogenesis and response to hypoxia, and provides new diagnostic and therapeutic strategies for diseases involving these mechanisms, as well as new approaches for research, selection, optimisation or production of compounds that exhibit activity against these diseases.

The present application relates therefore to the use of this protein, or its isoforms, as a target for the development of new molecules or methods for diagnosis. It also relates to compounds that are able to regulate HAI-2 protein and their therapeutic use. It is particularly adapted to the diagnosis, monitoring or treatment of cancers, diabetic retinopathy, macular degeneration, atherosclerosis, arthritis, rheumatoid arthritis, psoriasis, or diseases associated with delayed healing.

It is a primary object of the invention to provide variants of the HAI-2 protein (typically in an isolated or purified form), as well as specific fragments thereof. More particularly, the invention concerns a HAI-2 protein comprising the sequence of SEQ ID No 4 or 6.

It is another object of the invention to provide nucleic acids encoding variants of the HAI-2 protein (typically in an isolated or purified form), as well as specific fragments thereof. The nucleic acids can be DNA or RNA, for example. More particularly, the invention concerns nucleic acids encoding a HAI-2 protein comprising the sequence of SEQ ID No 4 or 6. More preferably, said nucleic acids comprises the sequence of SEQ ID No 3 or 5.

It is another object of the invention to provide a composition comprising a variant of the HAI-2 protein (typically in an isolated or purified form), or a specific fragment thereof, or a nucleic acid encoding such a variant or fragment.

It is another object of the invention to provide a method for treating a subject for a disease as defined herein above, particularly a disease with a component connected with angiogenesis, comprising administering an effective quantity of an HAI-2 protein inhibitor to an affected subject. In a preferred embodiment, the HAI-2 inhibitor is specific of one of the HAI-2 isoforms, more particularly wild type isoform or isoform 2.

It is another object of the invention to provide compounds that are HAI-2 protein inhibitors, particularly antibodies, inhibitory oligonucleotides (i.e., antisense sequence and a RNAi) or cytotoxic ligands, as well as compositions containing the same.

The invention also relates to a method for detecting the presence (or absence), predisposition to or for monitoring the stage of progression of a disease involving angiogenic phenomena or resulting from hypoxia, comprising determining the presence, quantity (relative or absolute) or distribution, in a sample from a subject, of an HAI-2 protein, its expression, altered forms thereof or of the gene or corresponding messenger RNA or altered forms thereof.

The invention also relates to methods for selecting, screening, characterising, optimising or producing active compounds, comprising a step of determining the capacity of a test compound to interact with the HAI-2 protein or the gene, to modulate its expression or to modulate its activity. In particular, the candidate compounds may be selected for their ability to specifically modulate or bind to one of the isoforms of HAI-2, for example wild type isoform or isoform 2.

The invention is particularly adapted to the development of medicaments or pharmaceutical compositions or methods for diagnosing or screening diseases that have a component connected with angiogenesis, as indicated herein above.

It is an object of the invention to use an inhibitor of the wild type isoform of HAI-2 or an activator of the isoform 2 of HAI-2 for preparing a medicament for inhibiting angiogenesis. Said inhibitor of the wild type isoform of HAI-2 can be an inhibitory oligonucleotide targeting exon 2, and/or junctions between exons 1-2 (SEQ ID No 8) and exons 2-3 (SEQ ID No 9) or an antibody (or any fragment or derivative thereof) which specifically binds a polypeptide encoded by exon 2 and/or junctions between exons 1-2 (SEQ ID No 13) and exons 2-3 (SEQ ID No 14). Preferably, this medicament is used for treating a disease selected from the group consisting of cancer, diabetic retinopathy, macular degeneration, atherosclerosis, arthritis, rheumatoid arthritis, psoriasis and diseases associated with delayed healing.

It is another object of the invention to use an activator of the wild type isoform of HAI-2 or an inhibitor of the isoform 2 of HAI-2 for preparing a medicament for activating angiogenesis. Said inhibitor of the isoform 2 of HAI-2 can be an inhibitory oligonucleotide or polynucleotide targeting the junction between exons 1-3 (SEQ ID No 10) or an antibody (or any fragment or derivative thereof) which specifically binds a polypeptide encoded by the junction between exons 1-3 (SEQ ID No 15). Preferably, this medicament is used for promoting wound healing, for treating ischaemia, or for preserving or restoring tissue integrity.

The invention also relates to an antibody (or any fragment or derivative thereof) which specifically binds an isoform of HAI-2 selected from the group consisting of the wild type isoform, isoform 2, isoform 3 and a combination of two isoforms. Preferably, said antibody (or any fragment or derivative thereof) specifically binds a polypeptide selected from the group consisting of SEQ ID Nos 13-17 and the polypeptide encoding by exon 2 or 3. More preferably, said antibody specifically binds a polypeptide selected from the group consisting of SEQ ID Nos 13, 15 and 17. The invention also contemplates a pharmaceutical composition compring an antibody according to the present invention.

The invention further concerns an inhibitory oligonucleotide specifically targeting one isoform of HAI-2 selected from the group consisting of the wild type isoform, isoform 2, and isoform 3. Preferably, said inhibitory oligonucleotide is targeting exon 2, exon 3, or the junction between exons 1-2 (SEQ ID No 8), exons 2-3 (SEQ ID No 9), exons 1-3 (SEQ ID No 10), exons 3-4 (SEQ ID No 11) or exons 2-4 (SEQ ID No 12). More preferably, said inhibitory oligonucleotide is targeting the junction between exons 1-3 (SEQ ID No 10), exons 2-3 (SEQ ID No 9), or exons 2-4 (SEQ ID No 12). The invention further contemplates a pharmaceutical composition comprising an inhibitory oligonucleotide according to the present invention. The term "targeting" indicates that the oligonucleotide comprises a sequence that is complementary to the selected region and is capable of specifically hybridising to said region.

The invention additionally concerns a nucleic acid probe or primer which specifically hybridises under stringent conditions with a sequence selected from the group consisting of exon 2, exon 3, or the junction between exons 1-2 (SEQ ID No 8), exons 2-3 (SEQ ID No 9), exons 1-3 (SEQ ID No 10), exons 3-4 (SEQ ID No 11) or exons 2-4 (SEQ ID No 12). More particularly, the nucleic acid probe or primer according to claim 20, wherein said nucleic acid probe or primer specifically hybridises under stringent conditions with a sequence selected from the group consisting of the junction between exons 1-3 (SEQ ID No 10), exons 2-3 (SEQ ID No 9), or exons 2-4 (SEQ ID No 12).

### PRODUCTS AND DEFINITIONS

### HAI-2 protein

In the context of the invention, the term HAI-2 protein denotes any polypeptide comprising the sequence of SEQ ID NO: 2 (wild type isoform), a variant of sequence SEQ ID NO: 2, or a fragment thereof.

The term "variant" particularly denotes any natural variants of the sequence SEQ ID NO: 2, resulting for example from polymorphism(s), splicing, mutation(s), etc. Such natural variants can therefore contain one or several mutations or substitutions, a deletion of one or several residues, etc. with respect to the sequence SEQ ID NO: 2. In this context, the present invention now describes the identification of new isoforms of the human HAI-2 protein, resulting from qualitative alterations at the RNA level, particularly from splicing. Thus, the applicants have demonstrated two new splice isoforms, the features of which are summarised below:
Isoform 2: presence of a first alternatively spliced exon labelled 2, situated between exon 1 and exon 3. The nucleic and amino acid sequences of this isoform is presented in SEQ ID NO: 3 and 4, respectively. The deleted region resulting from the deletion of exon 2 corresponds to residues 406 to 577 inclusive on SEQ ID NO: 2. At the amino acid level this results in an in-frame deletion of 57 amino acids.
Isoform 3: presence of a second alternative exon labelled 3, situated between exon 2 and exon 4. The nucleic and amino acid sequences of this isoform is presented in SEQ ID NO: 5 and 6. The deleted region resulting from the deletion of exon 3 corresponds to residues 578 to 637 inclusive on SEQ ID NO: 2. At the amino acid level this results in an in-frame deletion of 20 amino acids.

These isoforms represent particular objects of the invention. Thus; one aspect of the invention relates to a polypeptide comprising a sequence chosen from among the sequences SEQ ID NO: 2, 4 and 6, or a specific fragment of these sequences.

The term "specific" fragment or part denotes a fragment that is characteristic of the variants under consideration, typically a fragment with at least one genetic change that is characteristic of the variants in question. Such specific fragments differ therefore from the wild type sequence by the presence of a particular structural feature (e.g., a mutation, a new junction, retention of an intron, deletion of a sequence, a stop codon, a new sequence resulting from a reading frame shift, etc.) resulting from an alteration event demonstrated by the applicants. This particular structural feature is also denoted by the expression "target sequence". Specific fragments according to the invention therefore comprise at least one target sequence as defined herein above. The preferred fragments include at least 5 consecutive nucleotides from the sequence under consideration, preferably at least 8, more preferably at least 12. The fragments can contain up to 50, 75 or 100 nucleotides, or even more.

It is a particular object of the invention to particularly provide a polypeptide comprising a sequence selected from the group consisting of residues 21 to 51 (SEQ ID NO 13) of the sequence SEQ ID NO: 2, residues 21 to 51 (SEQ ID NO 15) of the sequence SEQ ID NO: 4, residues 78 to 108 (SEQ ID NO 14) of sequence SEQ ID NO: 2, residues 78 to 108 (SEQ ID NO 17) of sequence SEQ ID NO: 6 and residues 98 to 128 (SEQ ID NO 16) of the sequence SEQ ID NO: 2, or a fragment of these comprising at least five consecutive residues, preferably at least eight, more preferably at least twelve. Preferably, said fragment comprises at least one amino acid at either side of the junction between two exons, namely residues 36-37, 93-94 or 103-104 of sequence SEQ ID NO: 2, 4, or 6. More preferably, said fragment comprises at least two amino acids at either side of the junction between two exons, namely residues 35-38, 92-95 or 102-105 of sequence SEQ ID NO: 2, 4, or 6. Optionally, said fragment can comprise at least three amino acids at either side of the junction between two exons, namely residues 34-39, 91-96 or 101-106 of sequence SEQ ID NO: 2, 4, or 6.

The term variant denotes also the HAI-2 polypeptides that originate from another species, for example a rodent, a bovine, etc. Advantageously, the term HAI-2 protein denotes a polypeptide of human origin.

The term "variant" also includes any synthetic variant of an HAI-2 protein, and particularly any polypeptide containing one or several mutations, deletions, substitutions and/or additions of one or several amino acids with respect to the sequences SEQ ID NO: 2, 4 or 6. Preferably, it relates to polypeptides recognised by a polyclonal antibody produced using the HAI-2 protein from sequences SEQ ID NO: 2, 4 or 6.

The preferred synthetic variants advantageously share at least 75% identity with the primary sequences SEQ ID NO: 2, 4 or 6, preferably at least 80%, more preferably at least 85%. Still more preferably, the preferred synthetic variants share at least 90% identity with the primary sequences SEQ ID NO: 2, 4 or 6, preferably at least 95%, 96%, 97% or 98%. The degree of identity can be determined by various methods and by means of software known to professionals, such as, for example, the CLUSTAL method.

Some particular variants have a mutation or a substitution affecting at most 5 amino acids of the sequences SEQ ID NO: 2, 4 or 6.

Typically, the variants according to the invention are polypeptides that conserve at least one immunological, biological or pharmacological property of the HAI-2 protein from sequences SEQ ID NO: 2, 4 or 6. Their biological properties are particularly the inhibition of hepatocyte growth factor activator (HGFA), or the modulation of angiogenesis. Their immunological properties are for example the capacity to produce antibodies recognising the protein from SEQ ID NO: 2, 4 or 6.

As indicated, the term HAI-2 protein also includes polypeptides comprising fragments from the sequence SEQ ID NO: 2 or from a variant thereof. This more particularly concerns polypeptides comprising part of the sequences SEQ ID NO: 2, 4 or 6.

The polypeptide fragments of the invention preferably contain less than 200 amino acids, more preferably less than 180 amino acids, still more preferably less than 150 amino acids, for example less than 120 amino acids. They advantageously contain at least five consecutive residues from the HAI-2 protein sequence. Advantageously, the polypeptide fragments contain at least one region or a functional domain of the HAI-2 protein from sequence SEQ ID NO: 2, such as for example a Kunitz-type domain, a signal peptide sequence, a transmembrane domain, an epitope, a secondary structure (loop, sheet, etc), a consensus site, a junction between two exons, etc.

The preferred fragments of the invention comprise less than 200 amino acids from the sequences SEQ ID NO: 2, 4 or 6 and include at least one specific antigenic or immunogenic domain thereof. The present application proposes the production of polypeptide fragments of HAI-2, preferably including an immunogenic portion of HAI-2, which can be used to produce antibodies, assays, etc. These polypeptides can be developed using algorithms that enable the hydrophilicity or antigenicity of a polypeptide to be evaluated. According to the invention, the term immunogenic "fragment" or "portion" denotes any portion of the polypeptide containing an epitope, preferably a T or B cell epitope. Such a fragment therefore advantageously contains at least 7 consecutive amino acids from the sequences SEQ ID NO: 2, 4, or 6, more preferably at least 10 consecutive amino acids from the sequence under consideration, still more preferably at least 15 consecutive amino acids.

The particular fragments according to the invention retain one or several properties of HAI-2 such as those mentioned herein above. Such biologically active variants or fragments can be used to mimic the activity of HAI-2 or to produce corresponding antibodies.

Other particular fragments according to the invention are polypeptides capable of acting as an antagonist of the activity of HAI-2. Such fragments can be used to inhibit the action of HAI-2.

The HAI-2 polypeptides or proteins of the invention can contain heterologous residues added to the indicated amino acid sequence. Thus, it is an object of the invention to provide a polypeptide comprising all or part of the sequences SEQ ID NO: 2, 4, or 6, or a variant thereof, and a heterologous part.

The heterologous part can comprise amino acids, lipids, sugars, etc. It can also be a chemical, enzymatic, radioactive, etc. group(s). The heterologous part can in particular constitute a marker, a targeting agent, a stabilising agent, an agent that increases immunogenicity or facilitates production, a protective agent, an agent that facilitates penetration of the polypeptide into cells, a toxin, or an active compound, an antibody, etc.

The HAI-2 proteins of the invention can be presented in a soluble or purified form, or complexed to a carrier molecule such as KLH or serum albumin, or any other inert molecule (for example synthetic), such as a bead, for example. The polypeptides according to the invention are preferably free of contamination with proteins that are naturally present in their natural environment. In a particular embodiment, the polypeptides are coupled to a carrier molecule, notably for manufacturing antibodies. The coupling can be performed according to conventional techniques. The polypeptides can also be conjugated or fused to any other polypeptide or peptide molecule, such as, for example a peptide, polypeptide or a biologically active protein.

The HAI-2 proteins of the invention can be manufactured by any technique known per se to professionals, particularly by any chemical, biological, genetic, enzymatic, etc. technique, alone or in combination(s). The preferred methods comprise expressing a corresponding nucleic acid in an appropriate cell host, or performing artificial synthesis according to conventional techniques, such as solid phase synthesis.

The HAI-2 proteins according to the invention can be used in tests for selecting or screening, in assay methods, to regulate the activity of HAI-2 in vitro or in vivo, to produce medicinal products or vaccines, to target molecules, to produce anti-HAI-2 antibodies, particularly therapeutic antibodies, etc.

### HAI-2 gene

According to the invention, by "HAI-2 gene" is meant any nucleic acid encoding an HAI-2 protein as defined herein above. It can pertain to DNA or RNA, of natural or synthetic origin. It can particularly pertain to genomic, complementary, chimeric, or artificial DNA, or mRNA, etc. The HAI-2 gene can be in a single-stranded or doublestranded form. It can be obtained by any technique known to professionals starting from the sequence SEQ ID NO: 1 and the information contained within the present application. A particular HAI-2 gene comprises all or part of the sequences SEQ ID NO: 1, 3, or 5 or a complementary sequence, or a sequence that is different due to the degenerate nature of the genetic code, or a sequence hybridising to these under highly stringent conditions and encoding an HAI-2 protein. More particularly, by "stringent conditions" refers herein to hybridization at 65 °C. in a hybridization buffer consisting of 250 mmol/l sodium phosphate buffer pH 7.2, 7% (w/v) SDS, 1% (w/v) BSA, 1 mmol/l EDTA and 0.1 mg/ml single-stranded salmon sperm DNA.

The HAI-2 gene of the invention can be incorporated into a cloning or expression vector, in the genome of a cell, etc.

Examples of vectors that may be used to clone an HAI-2 gene of the invention are particularly plasmids, cosmids, episomes, artificial chromosomes, viruses, phages, etc.

Various commercial plasmids can be cited, such as pUC, pcDNA, pBR, etc. Among the viral vectors, the retroviruses, adenoviruses, AAV, herpes virus, etc. can be cited. Such vectors constitute particular objects of the invention.

The gene or vector can be introduced into a host cell, in order to produce an HAI-2 protein. Examples of cells that may be used are particularly mammalian, yeast, plant, insect or bacterial cells. Primary cells or cell lines established from mammalian cells can be mentioned. Hepatocytes, fibroblasts, endothelial cells or cell lines such as MDCK, HepG2, CHO, Vero, 293, etc. can be cited as examples of mammalian cells. Among the bacterial or yeast cells, *E. coli,* Saccharomyces and Kluyveromyces, for example, can be particularly cited. Such recombinant cells constitute particular objects of the invention.

### Nucleic acid probe

It is a particular object of the invention to provide a nucleic acid probe that can be used to detect an HAI-2 gene or a corresponding RNA in a sample, typically by selective hybridisation. Generally, the probe comprises the sequence of an HAI-2 gene as defined herein above or a part thereof. A probe of the invention typically comprises from 10 to 1000 nucleotides, preferably from 20 to 800, more preferably from 50 to 600, and is generally single-stranded. A particular example of a probe is an oligonucleotide that is specific for and complementary to at least one region of an HAI-2 gene or its corresponding RNA. The oligonucleotide is typically single-stranded, and generally has from 10 to 100 bases. Another example of a nucleic acid probe of the invention comprises the sequences SEQ ID NO: 1, 3, or 5, or a coding part thereof.

A particular probe according to the invention is a probe specific for a sequence SEQ ID NO: 1, 3, or 5 and capable of distinguishing between the different isoforms of HAI-2. It can typically be a probe that is specific for and complementary to exons specific to each of these isoforms or junction regions created by the presence or absence of alternative exons. For example, a probe specific for a junction between exons 1 and 3 is specific of the HAI-2 isoform 2. An example of such a probe can be a nucleic acid comprising the sequence SEQ ID 10 or a fragment thereof comprising at least 5 nucleotides at either side of the junction. A probe specific for a junction between exons 2 and 3 is specific of the HAI-2 wild type isoform. An example of such a probe can be a nucleic acid comprising the sequence SEQ ID 9 or a fragment thereof comprising at least 5 nucleotides at either side of the junction. A probe specific for a junction between exons 2 and 4 is specific of the HAI-2 isoform 3. An example of such a probe can be a nucleic acid comprising the sequence SEQ ID 12 or a fragment thereof comprising at least 5 nucleotides at either side of the junction. A probe specific for a junction between exons 1 and 2 is specific of the HAI-2 wild type and 3 isoforms. An example of such a probe can be a nucleic acid comprising the sequence SEQ ID 8 or a fragment thereof comprising at least 5 nucleotides at either side of the junction. A probe specific for a junction between exons 3 and 4 is specific of the HAI-2 wild type and 2 isoforms. An example of such a probe can be a nucleic acid comprising the sequence SEQ ID 11 or a fragment thereof comprising at least 5 nucleotides at either side of the junction.

The nucleic acid probes according to the invention can be labelled, for example by means of radioactive, enzymatic, fluorescent, luminescent, etc. markers. The probes can be free or immobilised on a matrix (column, bead, slide, membrane, etc).

In this respect, it is a particular object of the invention to provide a product comprising at least one probe as defined herein above, immobilised on a matrix. The matrix can be solid, flat or otherwise, uniform or otherwise, such as for example nylon, glass, plastic, metal, fibre, a ceramic material, silica, a polymer, etc., or any other compatible material. The probe is preferably immobilised by one end, under conditions that render the molecule accessible for a hybridisation reaction. Techniques for immobilising substances (such as nucleic acids, polypeptides, antibodies, etc.) on matrices have been described in the literature, and particularly in applications or patents n^{os} EP619 321, WO91/08307, US4,925,785 and GB2,197,720.

### Specific primer

It is another object of the invention to provide a nucleic acid primer that enables the (selective) amplification of an HAI-2 gene or a (specific) part thereof. A primer according to the invention is advantageously composed of 3 to 50 bases, preferably 5 to 40 and still more preferably 6 to 35 bases. It is typically single-stranded. A particular primer is complementary to at least one region of the HAI-2 gene or its corresponding RNA.

A preferred embodiment lies in a primer composed of a single-stranded nucleic acid comprising from 6 to 50 nucleotides complementary to at least part of the sequences SEQ ID NO: 1, 3, or 5 or their complementary strand.

Another preferred embodiment lies in a primer composed of a single-stranded nucleic acid comprising from 6 to 50 nucleotides that is complementary to a region bordering the sequences SEQ ID NO: 1, 3, or 5 and that enables amplification of at least part of this sequence. The term "bordering" indicates that the region is situated at preferably less than 300 bp from the sequence under consideration, more preferably less than 200 bp, still more preferably less than 150 bp. The primer can thus be complementary to and specific for a region bordering an HAI-2 gene in a genome, or bordering a coding sequence in a genome or an RNA molecule.

A particular primer according to the invention is a primer that is specific for a sequence SEQ ID NO: 1, 3, or 5, and capable of distinguishing between the different isoforms of HAI-2. Typically, it can be a probe specific for and complementary to (or enabling specific amplification of) all or part of an exon specific to each of these isoforms or new junction regions created by the presence or absence of alternative exons. The embodiments disclosed in the previous section are also relevant for a primer according the present invention.

The invention relates also to a primer pair comprising a sense sequence and a reverse sequence, wherein the primers of said pair hybridise to a region of an HAI-2 gene and enable amplification of at least a portion thereof. The primers can be complementary to part of the coding region of the HAI-2 gene, or regulatory regions (promoter, terminator sequence, etc).

### Antibodies

Another object of the invention encompasses the use of antibodies that are specific for HAI-2 protein or polypeptide or altered forms (splice variants) (i.e., capable of binding, preferably in a selective fashion, to such a polypeptide or protein or splice variant) for use as a diagnostic test for hypoxically related pathologies. These pathologies include cancers, retinopathies (particularly diabetic retinopathy and macular degeneration), atherosclerosis, arthritis, rheumatoid arthritis, psoriasis, as well as diseases associated with delayed healing, among others. The antibodies can be polyclonal or monoclonal. They can also be antibody fragments and derivatives with substantially the same antigen specificity, in particular antibody fragments (e.g., Fab, F(ab')2, CDRs), humanised, multifunctional, single chain (ScFv), etc. antibodies. The antibodies can be produced using conventional methods, comprising immunising a non-human animal with an HAI-2 protein or a fragment thereof including an epitope and collecting the serum (polyclonal) or spleen cells (in order to produce hybridomas by fusion with appropriate cell lines).

Different methods for producing polyclonal antibodies using various species have been described in prior art. Typically, the antigen is combined with an adjuvant (e.g. Freund's adjuvant) and administered to an animal, typically by subcutaneous injection. Repeated injections can be performed. Blood samples are collected and the immunoglobulin or serum is isolated.

The classic methods for producing monoclonal antibodies comprise immunising a non-human animal with an antigen, followed by recovery of spleen cells, which are then fused with immortalised cells, such as myeloma cells. The resulting hybridomas produce monoclonal antibodies and can be selected by limiting dilution in order to isolate individual clones.

Fab or F(ab')2 fragments can be produced by protease digestion, according to conventional techniques.

The invention also relates to a method of antibody production, comprising injecting an HAI-2 protein as defined herein above (or an immunogenic fragment thereof) into a non-human animal and recovering the antibodies or antibody producing cells.

The preferred antibodies are antibodies that are specific for the HAI-2 protein, i.e., those having a higher affinity for the HAI-2 protein than for other antigens, even if nonspecific or lower affinity binding cannot be excluded.

In a first variant, the antibodies are capable of recognising all the isoforms of sequences SEQ ID NO: 2, 4, and 6. Such antibodies are therefore directed against specific epitopes of HAI-2 that are common to these isoforms, preferably encoded by the exons 1, 4-7.

Other particular antibodies according to the invention are specific for one isoform of the HAI-2 protein of sequences SEQ ID NO: 2, 4, or 6. In particular, the antibodies preferred according to the invention are antibodies that are specific for (or generated by immunisation with a polypeptide comprising) the following sequences:
- residues 21 to 51 (SEQ ID NO 13) of sequence SEQ ID NO: 2, which is specific for isoforms wild type and 3;
- residues 78 to 108 (SEQ ID NO 14) of sequence SEQ ID NO: 2, which is specific for wild type isoform;
- residues 98 to 128 (SEQ ID NO 16) of sequence SEQ ID NO: 2, which is specific for isoforms wild type and 2;
- residues 21 to 51 (SEQ ID NO 15) of sequence SEQ ID NO: 4, which is specific for isoform 2; and,
- residues 78 to 108 (SEQ ID NO 17) of sequence SEQ ID NO: 6, which is specific for isoform 3;
- a fragment of any of these comprising at least five consecutive residues, preferably at least eight, more preferably at least twelve.

Preferably, said fragment comprises at least one amino acid at either side of the junction between two exons, namely residues 36-37, 93-94 or 103-104 of sequence SEQ ID NO: 2, 4, or 6. More preferably, said fragment comprises at least two amino acids at either side of the junction between two exons, namely residues 35-38, 92-95 or 102-105 of sequence SEQ ID NO: 2, 4, or 6. Optionally, said fragment can comprise at least three amino acids at either side of the junction between two exons, namely residues 34-39, 91-96 or 101-106 of sequence SEQ ID NO: 2, 4, or 6.

Such antibodies can be used to discriminate between these different forms, to analyse their regulation in samples, and to specifically modulate the activity of only one of the forms.

The invention also pertains to hybridomas producing monoclonal antibodies described herein above and their utilisation for producing said antibodies.

The antibodies of the invention can be coupled to heterologous fragments such as toxins, markers, medicinal products or any other therapeutic agent, in a covalent or noncovalent fashion, either directly, or through coupling agents. The markers can be chosen from among radiolabels, enzymes, fluorescent agents, magnetic particles, etc. The toxins can be chosen from among diphtheria toxin, botulinum toxin, ricin, etc.

The antibodies of the invention can also be immobilised on a matrix, such as a bead, column, gel, chip, etc. In this respect, it is a particular object of the invention to provide a product comprising at least one antibody specific for an HAI-2 protein immobilised on a matrix. The matrix can be solid, flat or otherwise, uniform or not, such as for example nylon, glass, plastic, metal, fibre, a ceramic material, silica, a polymer, etc., or any other compatible material. The antibody is preferably immobilised by one end, under conditions that leave the molecule accessible for an immunological reaction.

### DIAGNOSTIC APPLICATIONS

The present application describes new procedures for detecting or measuring the level of the hypoxic regulated gene HAI-2. As hypoxia is associated with a number of pathological conditions, the monitoring of HAI-2 expression levels provides a diagnostic tool for the study of diseases involving angiogenic phenomena, such as cancer or other diseases including diabetic retinopathy, macular degeneration, atherosclerosis, arthritis, rheumatoid arthritis, psoriasis, or diseases associated with delayed healing. These diagnostic or detection methods typically comprise determining the presence, quantity (relative or absolute) or distribution, in a sample from a subject, of the HAI-2 protein, its expression, altered forms thereof or of the gene or its corresponding messenger RNA. More particularly, the method comprises the detection of the wild type HAI-2 isoform, more preferably the detection of an increase of this isoform, as an indication of the presence or predisposition to a disease involving angiogenesis, particularly cancers.

The determination can be performed using different techniques, such as immunologic reaction, sequencing, selective hybridisation and/or amplification. Methods that can be used to determine the presence of proteins (or protein domains) are based for example on immuno-enzymatic reactions, such as ELISA, RIA, EIA, etc. Techniques that can be used to determine the presence or the expression of genes or RNA are for example PCR, RT-PCR, the ligase chain reaction (LCR), the PCE technique or TMA ("Transcriptional Mediated Amplification"), gel migration, electrophoresis, particularly DGGE ("denaturing gel gradient electrophoresis"), etc.

In the case where an amplification step is performed, it is preferably achieved using a primer or a primer pair as defined herein above.

It is a particular object of the invention to use nucleic acids that are complementary to and specific for the HAI-2 gene for detecting, characterising or monitoring the progression of a disease involving angiogenic phenomena such as cancer and other diseases including diabetic retinopathy, macular degeneration, atherosclerosis, arthritis, rheumatoid arthritis, psoriasis, or diseases associated with delayed healing, preferably cancers, more particularly the formation of metastases. This detection can be performed using DNA chips or by the application of PCR using a biopsy or biological fluids such as blood (particularly the serum or plasma), urine, seminal fluid, etc.

The invention also pertains to the development and use of immunological tests containing one or several antibodies as described herein above, or fragments or derivatives thereof. These tests enable the presence and/or quantity of HAI-2 protein to be detected and/or measured in a biological sample, this quantity being correlated with a pathological disease process.

A particular method comprises contacting a sample taken from a subject with a nucleic acid probe as defined herein above, and demonstrating hybridisation.

Another particular method comprises contacting a sample taken from a subject with a primer or a primer pair as defined herein above, and demonstrating an amplification product.

Another particular method comprises contacting a sample taken from a subject with an antibody as defined herein above, and demonstrating an antigen-antibody complex. In a specific embodiment, the presence, quantity or distribution of an HAI-2 protein or altered form thereof is thus determined using an antibody or a fragment or derivative thereof. More preferably, the antibody, fragment or derivative thereof specifically binds an isoform of HAI-2 selected from the group consisting of the wild type isoform, isoform 2, isoform 3 and a combination of two isoforms.

As indicated herein above, the HAI-2 protein is involved in angiogenic mechanisms. Differential expression of this protein is observed in response to hypoxic stress. Detection or assay of this protein (or of altered forms) in a sample from a subject therefore constitutes an indicator of the presence of such deleterious events and of the severity of these diseases. It is particularly suited to detect the presence, severity or predisposition to cancer, particularly colon cancer.

In this regard, a specific object of this invention resides in a method of detecting the presence, predisposition to or the progression of colon cancer in a subject, comprising determining the presence or quantity of HAI-2 protein or altered forms thereof in a biological sample from said subject, using an antibody, or a fragment or derivative thereof, that specifically binds HAI-2 and/or altered forms thereof.

The methods of this invention can be implemented using various biological samples that contain nucleic acids or polypeptides, such as biological fluids, including blood, plasma Serum, seminal fluid, saliva, etc., tissue samples, biopsies, sputum, stool, etc. The sample may be obtained by any known method, including non invasive techniques, or from sample collections. The sample may be treated prior to being tested, such as by lysis, centrifugation, clarification, dilution, purification, etc., to render or improve availability of nucleic acids or proteins.

Several tests can be performed in parallel, using several samples and/or using several probes, primers and/or antibodies.

It is another object of the invention to provide a kit that can be used to carry out a procedure as defined herein above, comprising:
i. a pair of primers or a probe or an antibody as defined herein above, and
ii. the reagents required for amplification or a hybridisation or immunological reaction.

More preferably, a particular object of this invention resides in a kit for detecting the presence or quantity of HAI-2 protein or altered forms thereof in a biological sample, the kit comprising an antibody that specifically binds HAI-2 and/or altered forms thereof, and a container or a reagent for an immunologic reaction.

An other particular object of this invention is a kit for detecting the presence or quantity of HAI-2 RNA or altered forms thereof in a biological sample, the kit comprising a nucleic acid probe or primer which specifically hybridises under stringent conditions with a sequence of HAI-2 RNA, more preferably selected from the group consisting of exon 2, exon 3, or the junction between exons 1-2 (SEQ ID No 8), exons 2-3 (SEQ ID No 9), exons 1-3 (SEQ ID No 10), exons 3-4 (SEQ ID No 11) or exons 2-4 (SEQ ID No 12), and a container or a reagent for an amplification or hybridization reaction.

### THERAPEUTIC APPLICATIONS

The present application describes the identification of a new protein involved in angiogenesis. This protein is differentially expressed under hypoxic conditions. This protein is therefore involved in the regulation of angiogenesis and represents a particularly interesting target for therapeutic interventions for various diseases, particularly such as cancers or retinopathies.

Indeed, inhibition of HAI-2 can bring about the activation of HGF and the induction of mitotic and angiogenic responses: Whilst, activation of HAI-2 can inhibit the activation of HGF and therefore down regulated angiogenic and mitotic responses.

However, the regulation of HAI-2 presents a further aspect. Indeed, HAI-2 can be expressed through several isoforms, namely wild type isoform, isoform 2 and isoform 3. The wild type isoform presents anti-angiogenic properties whereas isoform 2 has angiogenic properties.

It is a particular object of the invention therefore to use an HAI-2 protein inhibitor or activator for preparing a medicament (or a pharmaceutical composition) for inhibiting or activating angiogenesis. More particularly, the present invention concerns the use of inhibitor or activator which are specific of a particular isoform of HAI-2 (i.e., which essentially do not affect an other isoform).

Therefore, the present invention concerns the use of an inhibitor of the wild type isoform of HAI-2 or an activator of the isoform 2 of HAI-2 for preparing a medicament for inhibiting angiogenesis. Preferably, the present invention concerns the use of an inhibitor of the wild type isoform of HAI-2.

Many diseases have been described to have a component or stage connected with the phenomenon of angiogenesis. Very many cancers, retinopathies (particularly diabetic retinopathy and macular degeneration), atherosclerosis, arthritis, rheumatoid arthritis, psoriasis, as well as diseases associated with delayed healing can be cited, among others. The invention therefore enables the control of the onset, progression or development of these diseases in patients.

In a preferred embodiment of the present invention, the medicament for inhibiting angiogenesis is used for treating a disease selected from the group consisting of cancer, diabetic retinopathy, macular degeneration, atherosclerosis, arthritis, rheumatoid arthritis, psoriasis and diseases associated with delayed healing. In a most preferred embodiment, the treated disease is a cancer.

The invention can be used to treat different types of cancer, particularly solid tumours, such as for example cancers of the breast, lung, prostate, colon, uterus, head-and-neck, brain (e.g. glioblastoma), skin, liver, bladder, etc. It is particularly suited to treat colon cancer.

Optionally, said inhibitor of the wild type isoform of HAI-2 is an inhibitory oligonucleotide or polynucleotide specific of the wild type isoform. Said inhibitor is preferably specific of the wild type isoform by comparison with the isoform 2. Therefore, said inhibitor will preferentially target (or be specific of) exon 2, and/or junctions between exons 1-2 (SEQ ID No 8) and exons 2-3 (SEQ ID No 9). In a preferred embodiment, the inhibitory oligonucleotide or polynucleotide targets the junction between exons 2-3 (SEQ ID No 9). By "target" is intended that the inhibitory oligonucleotide or polynucleotide hyrbidizes under stringent conditions with the targeted sequence, i.e., comprises a sequence that is complementary and specific for a targeted sequence, allowing hybridisation to occur, thereby resulting in a specific inhibition of transcription and/or translation.

By "inhibitory oligonucleotide or polynucleotide" is intended an oligonucleotide or polynucleotide able to decrease the production of the HAI-2 protein, for instance by decreasing the production of the mRNA, decreasing its stability or its translation. Said inhibitory oligonucleotide or polynucleotide can be an antisense, a ribozyme, a siRNA or a shRNA.

The inhibitor of the wild type isoform of HAI-2 can also an antibody (or a fragment or derivative thereof) which specifically binds a polypeptide encoded by exon 2 and/or junctions between exons 1-2 (SEQ ID No 13) and exons 2-3 (SEQ ID No 14).

It is another object of the invention to use an activator of the wild type isoform of HAI-2 or an inhibitor of the isoform 2 of HAI-2 for preparing a medicament for activating angiogenesis. Preferably, the present invention concerns the use of an inhibitor of isoform 2 of HAI-2.

In a preferred embodiment of the present invention, the medicament for activating or promoting angiogenesis is used for promoting wound healing, for treating ischaemia, or for preserving or restoring tissue integrity.

In a first variant, said inhibitor of the isoform 2 of HAI-2 is an inhibitory oligonucleotide or polynucleotide specific of isoform 2. Said inhibitor is preferably specific of isoform 2 by comparison with the wild type isoform. Said inhibitor preferentially targets the junction between exons 1-3 (SEQ ID No 10).

The inhibitor of isoform 2 of HAI-2 can also an antibody which specifically binds a polypeptide encoded by the junction between exons 1-3 (SEQ ID No 15).

The activator can be any molecule which increases the activity of HAI-2. The activator can also be intended as the providing of the relevant HAI-2 isoform, either as protein or as an expression vector encoding the relevant HAI-2 isoform.

It is another object of the invention to provide methods for treating the diseases mentioned herein above, comprising administering an effective quantity of an HAI-2 activators or inhibitors to a patient.

In the context of the invention, the term "treatment" denotes preventive, curative or palliative treatment, as well patient management (reducing suffering, improving life expectancy, slowing the disease progression), etc. The treatment can moreover be carried out in combination with other active agents or treatments (chemotherapy, radiotherapy, gene therapy, etc.). The treatments and medicinal products of the invention are particularly intended for humans. The activator compounds can be of a diverse nature.

Further aspects and advantages of the present invention are illustrated in the following examples and figures.

### LEGEND TO THE FIGURES

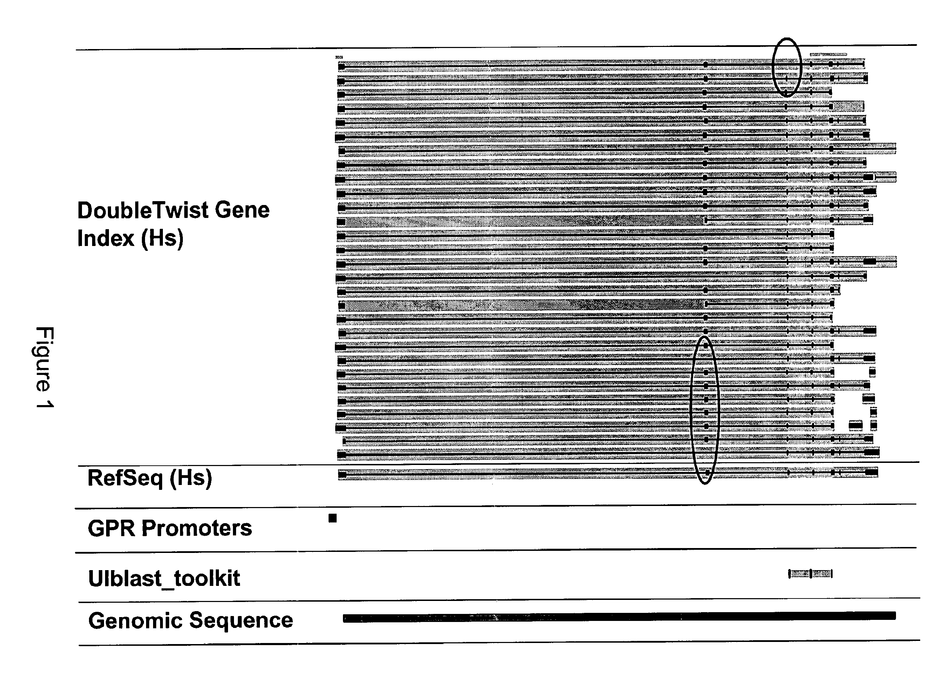

Figure 1: Representation of the HAI-2 gene and human alternative isoforms. A graphical view of alignment of the EXH-ARB0803-01 DATAS clone with genomic DNA and clusters of ESTs and mRNA (Prophecy software, DoubleTwist). The genomic sequences are shown as horizontal continuous bars. The dark box represents predicted promoter. The vertical grey line denotes the position of the DATAS fragment. Thin horizontal lines represent intronic sequences and thick boxes represent exons. The example shown demonstrates two exon skipping events (exon 2 and exon 3).

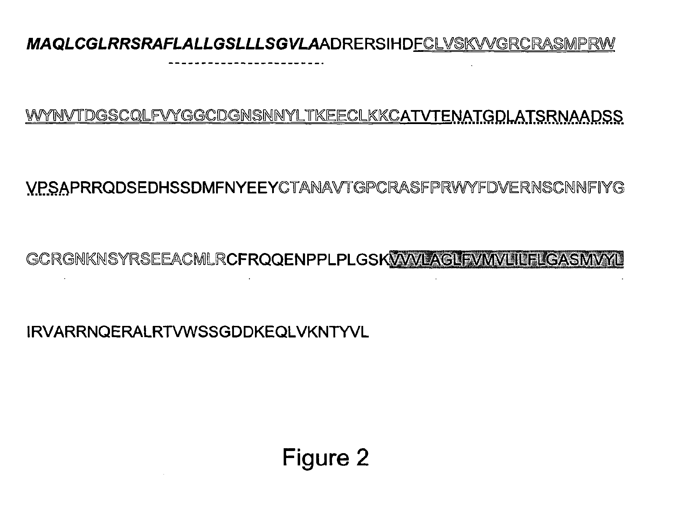

Figure 2: HAI-2 protein structural information demonstrating that the alternative splice events result in deletion of structural domains and that all isoforms retain the domain necessary for secretion of the protein. The signal peptide sequence is indicated in italic bold, the Kunitz-type domain in border letter (A), tand the transmembrane domain is indicated in grey box. The deleted amino acids sequences resulting from the two exon skipping events are underlined, in plain line for the skipping of exon 2 and in broken line for the skipping of exon 3, respectively.

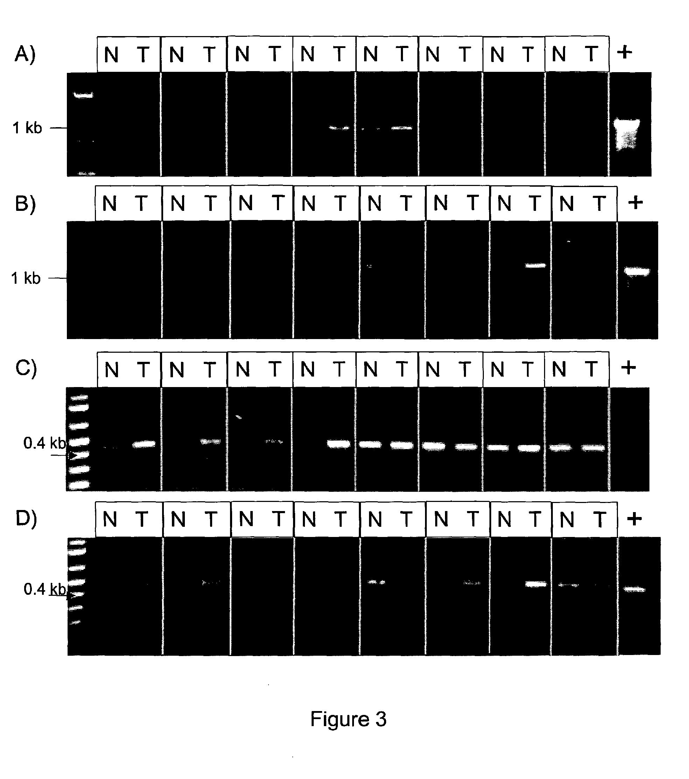

Figure 3: Expression profiling in colon tissues. Gel electophoresis of HAI-2 amplicons derived from :
   - A) 8 early stage tumors (T) with matched normal tissue (N) with HAI-2 F1/R1 primers. + is a PCR positive control.
   - B) 8 late stage tumors (T) with matched normal tissue (N) with HAI-2 F1/R1 primers. + is a PCR positive control.
   - C) 8 early stage tumors (T) with matched normal tissue (N) with HAI-2 F2/R2 primers. + is a PCR positive control.
   - D) 8 late stage tumors (T) with matched normal tissue (N) with HAI-2 F2/R2 primers. + is a PCR positive control.

### EXAMPLES

### 1. Materials And Methods Used

The entire molecular biology part (cloning, plasmid construction, transfection, establishment of stable clones, immunofluorescence, etc.) was performed using conventional protocols known to professionals.

The protein and nucleic acid sequences and are supplied in the list of sequences appended.

The RCC4 cell lines were cultured under normal conditions. The cells were cultured to between 70 and 80% confluence prior to treatment with normoxia (20% O₂, 16 hours) or hypoxia (0.1 % O₂, 16 hours). Total RNA was then extracted from these cells and used for both DATAS and expression profiling analyses.

### 2. Representation of the HAI-2 gene and human alternative isoforms

Expression profiling analysis using DATAS allowed the identification of a fragment of HAI-2 (SEQ ID NO:7) when comparing RNA from RCC4+VHL/N with RCC4-VHL/H. Bioinformatic analyses could identify two alternative splice events in the HAI-2 gene. The first results in skipping of exon 2 and the second in skipping of exon 3. The skipping of exon 2 give rise to an in frame deletion of 57 amino acids, and to the complete deletion of the first Kunitz-type domain. The first Kunitz-type domain of HAI-2 is responsible for the majority of the inhibitory activity of HAI-2 towards HGF/SF activator, at least in the human species (Qin, Denda et al. 1998; Kataoka, Itoh et al. 2002). Thus this alternative splice event may produce a protein which has a dominant negative effect. The skipping of exon 3 give rise to an in frame deletion of 20 amino acids between the two Kunitz-type domain. Both splice events result in in-frame deletions at the amino acid level that result in conformational alterations that will effect their ability to inhibit the activity of HGFA.

### 3. Demonstration of differential expression of HAI-2 in response to hypoxia and VHL status

A nucleic acid array displaying the clones generated by the RCC4-derived DATAS assay was constructed. This array includes the HAI-2 DATAS fragment ETH-ARCB0803 (SEQ ID NO:7). A significant differential expression of HAI-2 was demonstrated by macroarray profiling studies between RNA from -VHL and +VHL RCC4 cell lines (factor of 1.8). This differential expression was also observed when RNA from RCC4+VHL/N and RCC4-VHL/H were compared. and indicated that the HAI-2 DATAS fragment ETH-ARCB0803 (SEQ ID NO:7) is up-regulated in the hypoxic condition. These experiments confirm that;
1) HAI-2 is under the control of VHL and HIF1a gene products.
2) That HAI-2 is regulated by hypoxia.

### 4. Demonstration of the differential expression of HAI-2 in colon cancer tissues.

The expression of HAI-2 was measured by RT-PCR using RNAs extracted from colon cancer tissues. These tissues consisted in matched pairs of normal and cancer tissues derived from individual patients (ILS-Bio, Genomics Collaborative). They were classified as early stage for Duke's stages A and B and as late stage for Dukes' stages C and D.
Tissue RNA was isolated using Trizol™ on pulverized frozen tissue samples. RNA were analyzed on an Agilent 2100 analyzer as a quality control measure. Samples did not show any visible signs of degradation and peak ratios (area under the 18S and 28S ribosomal peaks) were greater than 1.5.

First strand cDNAs were generated through reverse transcription using oligo dT priming from a total of 32 individual RNA samples (8 matched early stage and 8 matched late stage). The first strand cDNAs were used as templates for a series of RT-PCR experiments after being normalised . A set of two primer pairs (described below) were used to analyze the expression of HAI-2. The PCR reactions were run using the following cycling profile:
initial melt 94 degrees (3 min):
   PCR cycle: melt 94 degrees (30 sec); annealing 55 degrees (30 sec); extension 72 degrees (1 minute).

Aliquots were removed at the completion of 20, 25 and 30 cycles and analyzed by gel electrophoresis. The primers used in the PCR reactions were:

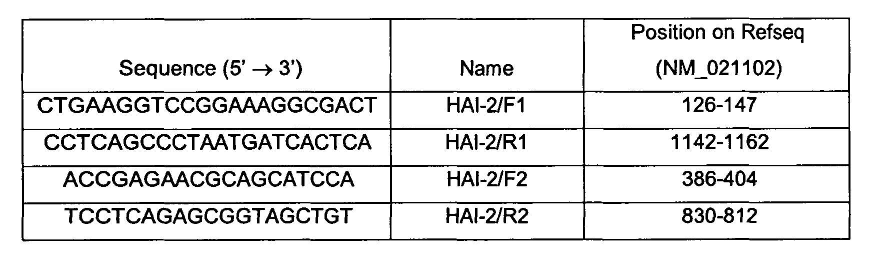

The results are consistent between the two sets of primers and clearly demonstrate that the HAI-2 RNA is overexpressed in 5 out of 8 early stage colon cancer samples and 5 out of 8 late stage colon cancer samples (figure 3). Consequently, HAI-2 proteins represent valid targets for the detection of colon cancer, specifically at early stages.

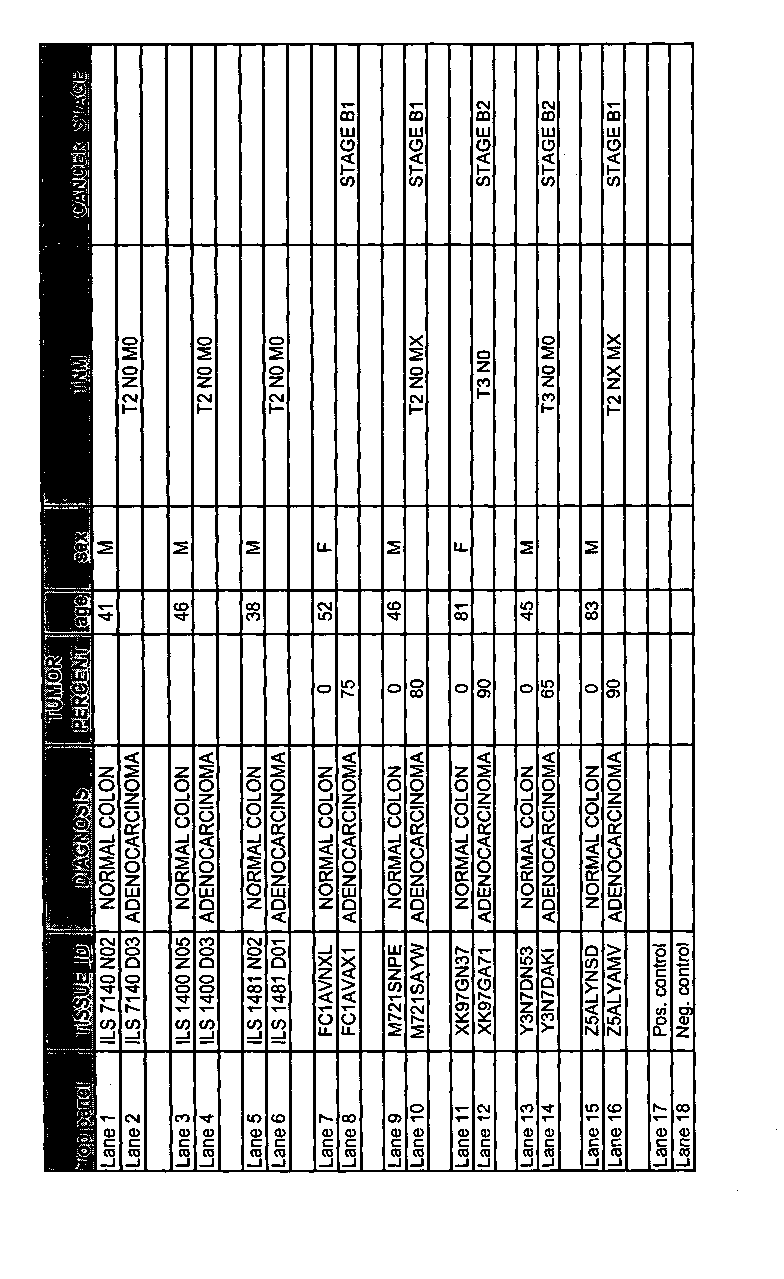

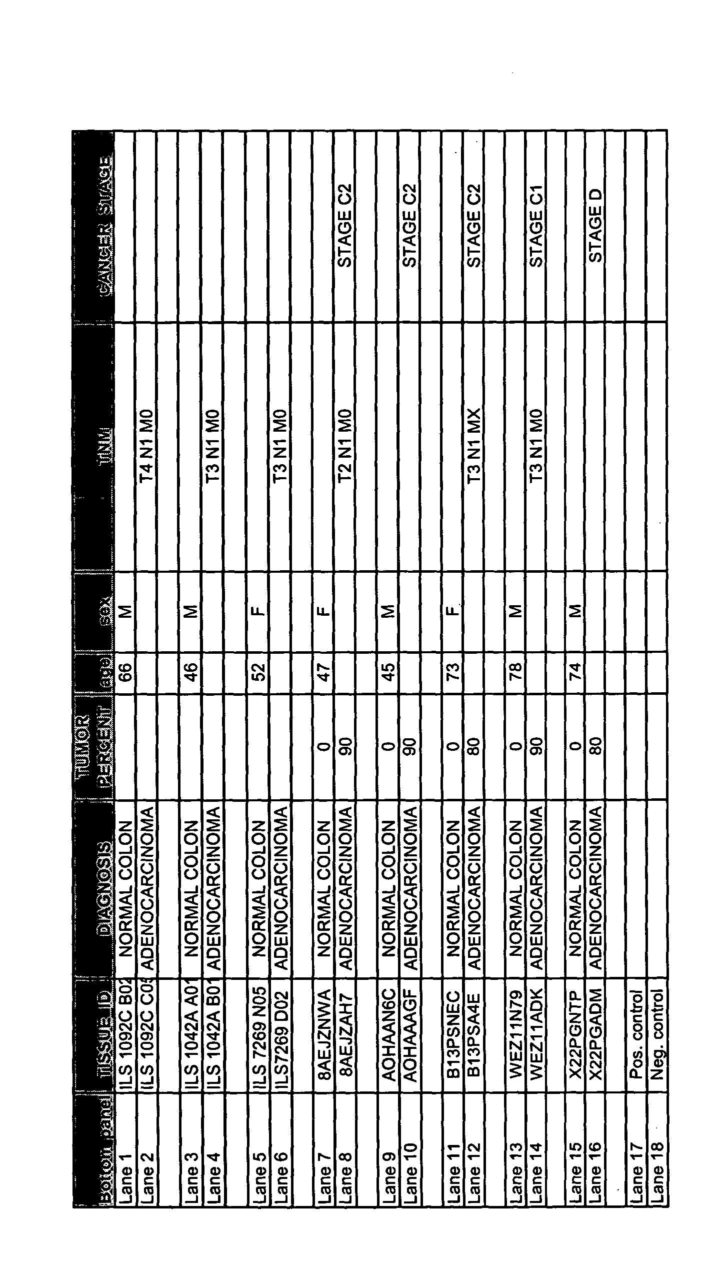

### Sequence definitions

SEQ ID NO:1 Full length HAI-2 nucleic acid sequence equivalent to NM_021102
SEQ ID NO:2 Full length HAI-2 amino acid sequence equivalent to NM_021102
SEQ ID NO:3 HAI-2 nucleic acid sequence of alternative splice event with exon 2 deleted
SEQ ID NO:4 HAI-2 amino acid sequence of alternative splice event with exon 2 deleted
SEQ ID NO:5 HAI-2 nucleic acid sequence of alternative splice event with exon 3 deleted
SEQ ID NO:6 HAI-2 amino acid sequence of alternative splice event with exon 3 deleted
SEQ ID NO:7 DATAS clone nulcelic acid sequence homologous to HAI-2
SEQ ID NO:8 Nucleic acid sequence at exon 1-2 junction (15bps either side of junction)
SEQ ID NO:9 Nucleic acid sequence at exon 2-3 junction (15bps either side of junction)
SEQ ID NO:10 Nucleic acid sequence at exon 1-3 junction (15bps either side of junction)
SEQ ID NO:11 Nucleic acid sequence at exon 3-4 junction (15bps either side of junction)
SEQ ID NO:12 Nucleic acid sequence at exon 2-4 junction (15bps either side of junction)
SEQ ID NO13 Amino acid sequence at exon 1-2 junction (15 amino acids either side of junction)
SEQ ID NO14 Amino acid sequence at exon 2-3 junction (15 amino acids either side of junction)
SEQ ID NO: 15 Amino acid sequence at exon 1-3 junction (15 amino acids either side of junction)
SEQ ID NO:16 Amino acid sequence at exon 3-4 junction (15 amino acids amino acids bps either side of junction)
SEQ ID NO:17 Amino acid sequence at exon 2-4 junction (15 amino acids either side of junction)

### References

Bussolino, F., M. F. Di Renzo, et al. (1992). "Hepatocyte growth factor is a potent angiogenic factor which stimulates endothelial cell motility and growth." J Cell Biol **119**(3): 629-41.
Delaria, K. A., D. K. Muller, et al. (1997). "Characterization of placental bikunin, a novel human serine protease inhibitor." J Biol Chem **272**(18): 12209-14.
Grant, D. S., H. K. Kleinman, et al. (1993). "Scatter factor induces blood vessel formation in vivo." Proc Natl Acad Sci U S A **90**(5): 1937-41.
Kataoka, H., H. Itoh, et al. (2002). "Mouse hepatocyte growth factor (HGF) activator inhibitor type 2 lacking the first Kunitz domain potently inhibits the HGF activator." Biochem Biophys Res Commun **290**(3): 1096-100.
Kawaguchi, T., L. Qin, et al. (1997). "Purification and cloning of hepatocyte growth factor activator inhibitor type 2, a Kunitz-type serine protease inhibitor." J Biol Chem **272**(44): 27558-64.
Qin, L., K. Denda, et al. (1998). "Functional characterization of Kunitz domains in hepatocyte growth factor activator inhibitor type 2." FEBS Lett **436**(1): 111-4.

B0277-HAI-2.ST25.txt
## Claims

1. A method for detecting the presence of or a predisposition to a disease involving angiogenic phenomena in a subject, or for monitoring the progression of the stage of development of a disease involving angiogenic phenomena, comprising determining the presence, quantity (relative or absolute) or distribution, in a sample from said subject, of HAI-2 protein, its expression, altered forms thereof or of the gene or corresponding messenger RNA.

2. A method according to claim 1, wherein the disease is a cancer, more specifically colon cancer.

3. The method of claim 1 or 2, wherein the sample is blood, plasma or serum.

4. The method of any one of claims 1 to 3, wherein the presence, quantity or distribution of said HAI-2 protein or altered forms thereof is determined using an antibody or a fragment or derivative thereof.

5. The method of claim 4, wherein the antibody, fragment or derivative thereof specifically binds an isoform of HAI-2 selected from the group consisting of the wild type isoform, isoform 2, isoform 3 and a combination of two isoforms.

6. A method of detecting the presence, predisposition to or the progression of colon cancer in a subject, comprising determining the presence or quantity of HAI-2 protein or altered forms thereof in a biological sample from said subject, using an antibody, or a fragment or derivative thereof, that specifically binds HAI-2 and/or altered forms thereof.

7. A kit for detecting the presence or quantity of HAI-2 protein or altered forms thereof in a biological sample, the kit comprising an antibody that specifically binds HAI-2 and/or altered forms thereof, and a container or a reagent for an immunologic reaction.

8. A kit for detecting the presence or quantity of HAI-2 RNA or altered forms thereof in a biological sample, the kit comprising a nucleic acid probe or primer which specifically hybridises under stringent conditions with a sequence of HAI-2 RNA, more preferably selected from the group consisting of exon 2, exon 3, or the junction between exons 1-2 (SEQ ID No 8), exons 2-3 (SEQ ID No 9), exons 1-3 (SEQ ID No 10), exons 3-4 (SEQ ID No 11) or exons 2-4 (SEQ ID No 12), and a container or a reagent for an amplification or hybridization reaction.

9. An antibody which specifically binds an isoform of HAI-2 selected from the group consisting of the wild type isoform, isoform 2, isoform 3 and a combination of two isoforms.

10. The antibody according to claim 9, wherein said antibody specifically binds a polypeptide selected from the group consisting of SEQ ID Nos 13-17 and the polypeptide encoding by exon 2 or 3.

11. The antibody according to claim 10, wherein said antibody specifically binds a polypeptide selected from the group consisting of SEQ ID Nos 13, 15 and 17.

12. HAI-2 protein comprising the sequence of SEQ ID No 4 or 6.

13. A nucleic acid encoding HAI-2 protein according to claim 12.

14. The nucleic acid according to claim 13, wherein said nucleic acid comprises the sequence of SEQ ID No 3 or 5.

15. A nucleic acid probe or primer which specifically hybridises under stringent conditions with a sequence selected from the group consisting of exon 2, exon 3, or the junction between exons 1-2 (SEQ ID No 8), exons 2-3 (SEQ ID No 9), exons 1-3 (SEQ ID No 10), exons 3-4 (SEQ ID No 11) or exons 2-4 (SEQ ID No 12).

16. The nucleic acid probe or primer according to claim 15, wherein said nucleic acid probe or primer specifically hybridises under stringent conditions with a sequence selected from the group consisting of the junction between exons 1-3 (SEQ ID No 10), exons 2-3 (SEQ ID No 9), or exons 2-4 (SEQ ID No 12).

17. Use of an inhibitor of the wild type isoform of HAI-2 or of an activator of the isoform 2 of HAI-2 for preparing a medicament for inhibiting angiogenesis, preferably for treating a disease selected from the group consisting of cancer, diabetic retinopathy, macular degeneration, atherosclerosis, arthritis, rheumatoid arthritis, psoriasis and diseases associated with delayed healing.

18. Use according to claim 17, wherein said inhibitor of the wild type isoform of HAI-2 is an inhibitory oligonucleotide targeting exon 2 and/or junctions between exons 1-2 (SEQ ID No 8) and exons 2-3 (SEQ ID No 9), or an antibody, fragment or derivative thereof, which specifically binds a polypeptide encoded by exon 2 and/or junctions between exons 1-2 (SEQ ID No 13) and exons 2-3 (SEQ ID No 14).

19. Use of an activator of the wild type isoform of HAI-2 or of an inhibitor of the isoform 2 of HAI-2 for preparing a medicament for activating angiogenesis, preferably for promoting wound healing, for treating ischaemia, or for preserving or restoring tissue integrity.

20. Use according to claim 19, wherein said inhibitor of the isoform 2 of HAI-2 is an inhibitory oligonucleotide or polynucleotide targeting the junction between exons 1-3 (SEQ ID No 10) or an antibody which specifically binds a polypeptide encoded by the junction between exons 1-3 (SEQ ID No 15).

21. An inhibitory oligonucleotide specifically targeting one isoform of HAI-2, wherein said oligonucleotide is targeting exon 2, exon 3, or the junction between exons 1-2 (SEQ ID No 8), exons 2-3 (SEQ ID No 9), exons 1-3 (SEQ ID No 10), exons 3-4 (SEQ ID No 11) or exons 2-4 (SEQ ID No 12).

22. Use according to claim 18 or 19, or inhibitory oligonucleotide according to claim 21, wherein said inhibitory oligonucleotide is selected from the group consisting of an antisense, a ribozyme, a siRNA and a shRNA.
